## Europäisches Patentamt

## European Patent Office

## Office européen des brevets

(19)

(11) Veröffentlichungsnummer: **0 101 007**
**B1**

(12)

# EUROPÄISCHE PATENTSCHRIFT

(45) Veröffentlichungstag der Patentschrift:
**11.05.88**

(21) Anmeldenummer: **83107635.1**

(22) Anmeldetag: **03.08.83**

(51) Int. Cl.⁴: **B 01 F 3/08,** B 01 F 5/08,
B 01 F 5/06, A 61 K 7/00,
A 61 K 9/10

(54) **Herstellung von pharmazeutischen oder kosmetischen Dispersionen.**

(30) Priorität: **14.08.82 DE 3230289**

(43) Veröffentlichungstag der Anmeldung:
**22.02.84 Patentblatt 84/8**

(45) Bekanntmachung des Hinweises auf die Patenterteilung:
**11.05.88 Patentblatt 88/19**

(84) Benannte Vertragsstaaten:
**AT CH DE FR GB IT LI**

(56) Entgegenhaltungen:
**EP - A - 0 045 008
DE - A - 2 633 288
DE - B - 1 757 111
DE - C - 401 477
GB - A - 966 336
GB - A - 2 002 652
GB - A - 2 063 695
US - A - 3 635 834
US - A - 4 135 829**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP
Patentabteilung, D-5090 Leverkusen 1 Bayerwerk (DE)**

(72) Erfinder: **Bücheler, Manfred, Dipl.-Ing., Lorkenhoehe 49,
D-5063 Overath (DE)**
Erfinder: **Gehringer, Hans, Heinering 45,
D-5000 Koeln 71 (DE)**
Erfinder: **Klinksiek, Bernd, Dipl.-Ing., Obervolbach 10,
D-5060 Bergisch Gladbach 4 (DE)**
Erfinder: **Koglin, Bernd Dr., Zehntweg 27,
D-5060 Bergisch Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von feinteiligen, stabilen, pharmazeutischen oder kosmetischen Dispersionen aus einer wässrigen Phase und einer in Wasser unlöslichen oder nicht vollständig löslichen organischen Phase (Ölphase), bei dem aus den beiden Phasen nach bekannten Emulgiermethoden zunächst eine Voremulsion hergestellt wird, die dann zum Endprodukt weiterverarbeitet wird. Ferner betrifft die Erfindung eine Vorrichtung zur Durchführung des Verfahrens.

Üblicherweise werden pharmazeutische oder kosmetische Emulsionen so hergestellt, dass man bei einer Temperatur von 60 bis 80°C die aufgeschmolzene organische Phase (Ölphase) und die gesamte, auf die gleiche Temperatur gebrachte wässrige Phase in einem Rührkessel vereinigt und bei Salben und Cremes in einem Salbenreaktor mit Rühr- und Homogenisierwerkzeugen auf Raumtemperatur abkühlt und dabei homogenisiert. Bei fliessfähigen Emulsionen wird die so erhaltene Rohemulsion voremulgiert und in einem Mantel- oder Durchflusskühler auf 20 bis 40°C abgekühlt und anschliessend mit einem Hochdruckhomogenisator feinstdispergiert. Ein Nachteil bei dieser Art der Herstellung einer fliessfähigen Emulsion liegt darin, dass der gesamte Ansatz zunächst aufgeheizt und nach dem Voremulgieren wieder abgekühlt werden muss, um dann die gesamte Emulsion mit häufig nur einem geringen Anteil an disperser Ölphase mittels Hochdruckhomogenisatoren auf die gewünschte Feinheit zu bringen. Hochdruckhomogenisatoren erfordern einen sehr hohen Betriebsdruck in der Grössenordnung von 200 bar, der nur mit relativ aufwendigen mehrstufigen Hochdruckkolbenpumpen mit hoher Antriebsleistung erzeugt werden kann. Darüber hinaus tritt ein hoher Energieverbrauch durch die oben beschriebene verfahrenstechnisch bedingte Temperaturführung auf.

Ein weiterer Nachteil besteht darin, dass durch die grossen Scherkräfte im Hochdruckhomogenisator hochmolekulare organische Verbindungen zerstört werden können, so dass Produktschädigungen auftreten.

Zur Herstellung von Salben- und Cremepräparaten werden bisher aufwendige Salbenreaktoren (schwere Maschinenständer, Vakuumeinrichtungen, komplizierte Wellenabdichtungen, etc.) benutzt, in denen durch das gleichzeitige Rühren, Homogenisieren und Abkühlen völlig undefinierte Herstellbedingungen vorherrschen (z.B. wird nur im statistischen Mittel jedes Volumenelement des Produktes den Scherspalt des Homogenisators durchlaufen). Ausserdem ergeben sich durch schlechten Wärmeübergang lange Kühlzeiten.

Der Erfindung liegt die Aufgabe zugrunde, die Wirtschaftlichkeit der bekannten Verfahren zu verbessern. Darunter ist hier zu verstehen, dass bei geringstmöglichem apparativem Aufwand auch eine günstige Energiebilanz angestrebt wird (Reduzierung der Investitions- und Betriebskosten).

Als Randbedingung ist dabei zu beachten, dass keine Produktschädigungen auftreten (zu hohe Scherbeanspruchung) und dass die Teilcheneigenschaften (mittlere Teilchengrösse und Teilchengrössenverteilung) die gleichen sind wie bei den bekannten Verfahren.

Diese Aufgabe wird, ausgehend von dem eingangs beschriebenen Verfahren, erfindungsgemäss dadurch gelöst, dass eine Voremulsion durch einen Strahldispergator mit den Abmessungen

a) 0,5 bis 0,8 mm Durchmesser und
b) Längen-/Durchmesser-Verhältnis von 1,5 bis 2 gepumpt wird und dabei annähernd die gesamte Pumpenenergie in der Dispergierzone verbraucht wird und die Dispersion als ungerichteter Strahl die Dispergierzone verlässt.

Vorteilhaft wird das erfindungsgemässe Verfahren mit einem Phasenumkehrverfahren kombiniert. Beim Phasenumkehrverfahren wird zur Herstellung der Voremulsion zunächst die Ölphase als äussere Phase vorgelegt und in diese die wässrige Phase als innere Phase einemulgiert. Bei der Voremulsion bildet also – im Gegensatz zum Endprodukt – die wässrige Phase die innere (disperse) Phase und die Ölphase die äussere (kontinuierliche) Phase. Die so hergestellte Voremulsion wird nun im nachfolgenden Verfahrensschritt durch den Strahldispergator gepumpt, wobei die Temperatur, das Mengenverhältnis der beiden Phasen und der Druck am Strahldispergator derart eingestellt werden, dass im Strahlendispergator gleichzeitig mit der Homogenisierung und Feindispergierung eine Phasenumkehr der Emulsion erfolgt. Beim Passieren des Strahldispergators wird also die wässrige Phase in die äussere Phase und die Ölphase in die innere Phase überführt. Die zu einem bestimmten Mengenverhältnis gehörende Phasenumkehrtemperatur lässt sich ohne weiteres empirisch ermitteln.

Die Anwendung des Phasenumkehrverfahrens in Kombination mit dem Strahldispergator führt zu einem sehr einfachen Dispergierverfahren. Würde z.B. zur Homogenisierung nach der Standardtechnik ein Homogenisierdruck von 200 bar bei Verwendung eines Hochdruckhomogenisators und 40 bis 50 bar bei Verwendung eines Strahldispergators benötigt, so reicht bei der Kombination von Strahldispergierung und Phasenumkehrverfahren bereits ein Betriebsdruck im Bereich von 2 bis 50 bar, vorzugsweise 10 bis 50 bar, der schon mit relativ einfachen und billigen Pumpen erzeugt werden kann.

Eine bevorzugte Ausführung der Erfindung besteht darin, dass die Phasenumkehr bei einer Konzentration von 50 bis 70 Gew.-% Ölphase (entsprechend 50 bis 30 Gew.-% wässriger Phase) durchgeführt wird. Dies bedeutet, dass bei der Strahldispergierung eine hochkonzentrierte Emulsion anfällt, die später auf die gewünschte Endkonzentration verdünnt wird. Die Verdünnung erfolgt durch Zugabe von kalter wässriger Phase, so dass gleichzeitig eine Abkühlung erfolgt. Da das Verfahren nur mit einem Bruchteil der gesamten Emulsionsmenge durchgeführt werden muss, erreicht man eine hohe Raum-Zeit-Ausbeute bei günstiger Energiebilanz. Dies bedeutet, dass man entweder mit geringerem Kesselvolumen die gleiche Menge im Vergleich zu den früher benutzten Verfahren oder bei entsprechend vergrösserter Verdünnerkesselkapazität in einem Ansatz eine wesentlich vergrösserte Produktmenge herstellen kann.

Kosmetische und pharmazeutische Dispersionen sind im allgemeinen aus folgenden Grundkomponenten zusammengesetzt:

1. Die wässrige Phase besteht aus einer wässrigen Lösung von Glyzerin, Glykol, kosmetischen oder pharmazeutischen Wirkstoffen, viskositätserhöhenden Zusätzen und Konservierungsmitteln.

2. Die organische in Wasser unlösliche oder nicht vollständig lösliche Ölphase besteht aus Glyzerin- oder Fettsäureester und/oder flüssigen oder halbfesten Kohlenwasserstoffen, höherwertigen Alkoholen, nicht-ionogenen Emulgatoren und fettlöslichen pharmazeutischen oder kosmetischen Wirkstoffen.

Bei der Voremulsion bildet die wässrige Phase die innere Phase und die Ölphase die äussere Phase, während bei dem fertigen Endprodukt die Ölphase als innere Phase in der wässrigen Phase feinverteilt dispergiert ist.

Gegenstand der Erfindung ist ferner eine Vorrichtung zur Durchführung des vorstehend beschriebenen Verfahrens mit einer ersten Emulgiervorrichtung zur Herstellung der Voremulsion und einer zweiten Emulgiervorrichtung zur Herstellung der fertigen Emulsion. Dabei besteht die zweite Emulgiervorrichtung aus einem Strahldispergator mit einer oder mehreren Düsen, deren Durchmesser 0,5 bis 0,8 mm und deren Verhältnis von Länge l/d zu Durchmesser 1,5 bis 2 beträgt.

Mit dieser Dimensionierung beträgt das Volumen der Dispergierzone nur 0,1 mm$^3$ bis 1 mm$^3$. Es wurde gefunden, dass annähernd die gesamte Pumpenergie in der Dispergierzone verbraucht wird und die Dispersion als ungerichteter Strahl die Dispergierzone verlässt.

Die Düsen sind entweder so angeordnet, dass der austretende Emulsionsstrahl auf eine feste Wand prallt oder die Emulsionsstrahlen selbst aufeinanderprallen. Bei der zuletzt genannten Ausführung werden die nach dem Austritt aus den Düsen noch vorhandenen Strömungsenergien durch das Zusammenprallen der Strahlen verbraucht.

Der wesentliche und überraschende Vorteil des erfindungsgemässen Verfahrens liegt darin, dass mit einer vereinfachten Apparatur eine verbesserte Raum-Zeit-Ausbeute erzielt wird. Von Bedeutung ist dabei, dass die gleiche Teilchenfeinheit bei dem Strahldispergator schon mit wesentlich geringerem Druck und folglich weniger aufwendigen Pumpen erreicht werden kann wie bei den früher verwendeten Hochdruckhomogenisatoren. Ausserdem ist das Verfahren produktschonend, da die hochmolekularen Bestandteile in der Ölphase und Wasserphase im Stahldispergator einer geringeren Scherbeanspruchung ausgesetzt werden.

Bei der Herstellung von Salben und Cremes sind die Prozessbedingungen im Gegensatz zu früher streng definiert. Insbesondere wird im Strahldispergator ein sehr enges Teilchengrössenverteilungsspektrum erzeugt. Daher ist die Modellübertragung vom Labor- in den Produktionsmassstab völlig unproblematisch.

Aus EP-A-45 008 ist bereits ein Verfahren zur Herstellung von feinteiligen, stabilen, pharmazeutischen und kosmetischen Dispersionen bekannt, und in DE-A-2 633 288 wurden Strahldispergatoren beschrieben.

Die erfindungsgemässen Abmessungen des Strahldispergators befinden sich zwar innerhalb der in den Entgegenhaltungen mitgeteilten Abmessungen des kreisförmigen Homogenisierungsquerschnittes; jedoch wurde unsererseits gefunden, dass der Strahldispergator dann optimal arbeitet, wenn der Durchmesser der Düse bei 1,5 bis 2 liegt. Nur mit diesen Düsenabmessungen lassen sich hochdisperse, stabile Emulsionen herstellen. Bei der Massstabvergrösserungen sind einzelne Düsen parallel zu schalten und nicht die Düsenquerschnittfläche zu vergrössern, wie dies in DE-A-2 633 288 mittels Irisblende bzw. Rechteckquerschnitt beschrieben ist.

Im folgenden wird die Erfindung anhand von Ausführungsbeispielen und Zeichnungen näher beschrieben. Es zeigen

Fig. 1 ein Fliessschema für das erfindungsgemässe Verfahren und

Fig. 2 bis 4 verschiedene Ausführungen (Querschnitt) des Strahldispergators.

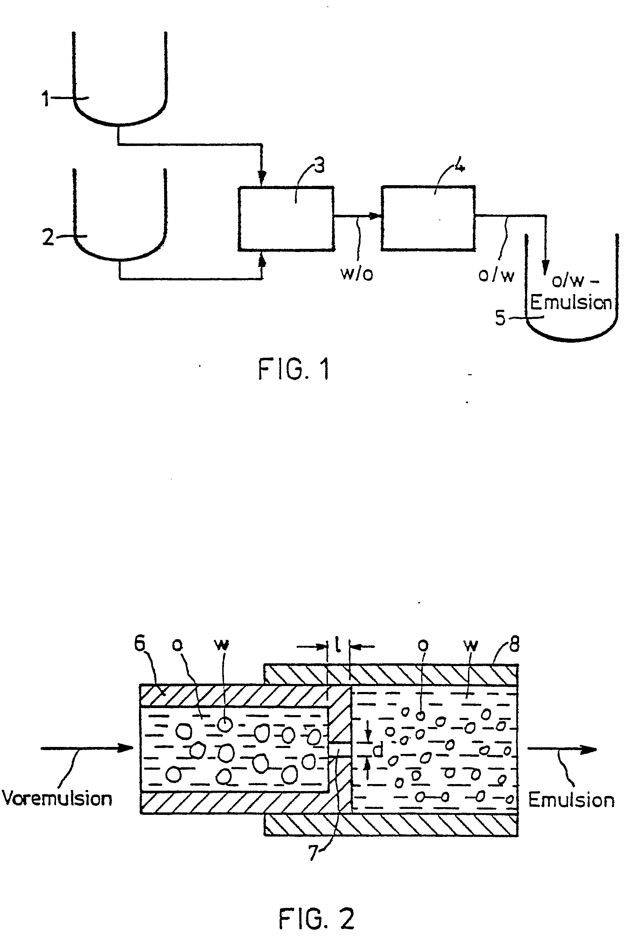

Gemäss Fig. 1 befindet sich die wässrige Phase in einem Kessel 1 und die Ölphase in einem Kessel 2. Die Kesseltemperaturen müssen gegebenenfalls durch Beheizung so eingestellt werden, dass die Temperatur der beiden Phasen oberhalb des Erstarrungspunktes eventuell vorhandener Fettbestandteile liegt. Die beiden Phasen werden dann im Rührkessel 3 zusammengerührt und voremulgiert. Die so hergestellte Voremulsion wird anschliessend aus dem Rührkessel 3 durch den Strahldispergator 4 gepumpt, in dem die für das Endprodukt charakteristische Homogenisierung und Feindispergierung erfolgt. Im Anschluss daran wird aus der hochkonzentrierten Emulsion durch Einleiten der wässrigen Phase in den Lagerkessel 5 die gewünschte Endkonzentration eingestellt.

(Verdünnung). Das Verfahren das hier diskontinuierlich ausgeführt wird, kann auch in einer kontinuierlichen Fahrweise betrieben werden. In diesem Fall werden beide Phasen aus den Kesseln 1 und 2 heraus kontinuierlich in den Rührkessel 3 dosiert, voremulgiert und anschliessend durch den Strahldispergator 4 gepumpt.

In vielen Fällen lässt sich leichter eine Wasser-in-Öl-Emulsion mit hoher Teilchenfeinheit erzeugen als eine Öl-in-Wasser-Emulsion. In solchen Fällen wird das vorstehend beschriebene Verfahren in der Weise modifiziert, dass im Strahldispergator 4 eine Phasenumkehr eintritt. Dabei geht man so vor, dass aus dem Kessel 2 zunächst die Ölphase im Rührkessel 3 vorgelegt wird und dann langsam die wässrige Phase eingemischt wird. Dadurch entsteht eine Voremulsion, bei der die Ölphase die äussere und die wässrige Phase die innere Phase bildet (W/O-Emulsion). Bei der anschliessenden Dispergierung und Homogenisierung im Strahldispergator 4 findet dann eine Phasenumkehr statt, derart, dass die äussere Phase zur inneren und die innere Phase zur äusseren Phase wird. Es entsteht eine Öl-in-Wasser-Emulsion (O/W-Emulsion). Massgebend für den Phasenumkehrpunkt sind die Konzentrationen der beiden Phasen (Mengenverhältnis), die Temperatur und die mechanische Beanspruchung im Strahldispergator 4. Für ein vorgegebenes Produkt kann der Phasenumkehrpunkt ohne weiteres empirisch als Funktion der oben erwähnten Verfahrensparameter bestimmt werden. Zur Bestimmung des Phasenumkehrpunktes können ferner physikalische Messmethoden, wie z.B. die Messung der elektrischen Leitfähigkeit oder der Ultraschallabsorption, herangezogen werden, da sich diese Grössen am Phasenumkehrpunkt sprunghaft ändern.

Im folgenden werden verschiedene technische Ausführungen des Strahldispergators 4 beschrieben. Die einfachste Ausführung eines Strahldispergators ist in Fig. 2 dargestellt. Er besteht aus einem Zulaufrohr 6, das an seinem Ende bis auf eine Kapillarbohrung 7 verschlossen ist und durch diese Kapillarbohrung mit einem Auslaufrohr 8 verbunden ist. Das Auslaufrohr 8 schliesst im übrigen dicht an das Zulaufrohr 6 an. Der Durchmesser d der Kapillarbohrung liegt z.B. bei 0,6 mm, ihre Länge l bei 1 mm. Durch systematische Versuchsreihen wurde festgestellt, dass man optimale Ergebnisse hinsichtlich der Teilcheneigenschaften und der Energiedissipation erhält, wenn folgende Dimensionierung eingehalten wird:

$$0,3\,\text{mm} \leq d \leq 1\,\text{mm},$$
$$1,5 \leq l/d \leq 2.$$

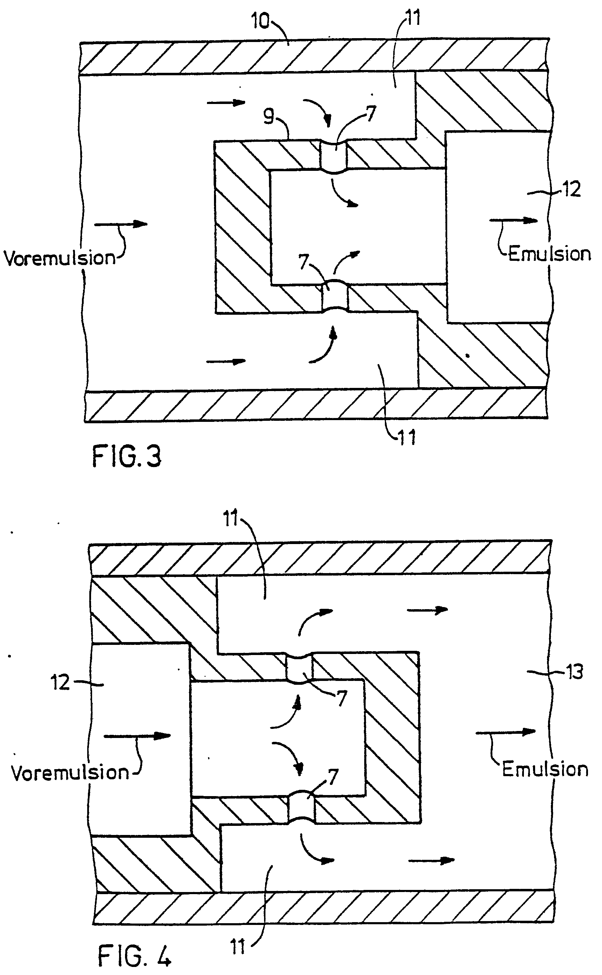

Eine alternative Ausführung des Strahldispergators ist in den Fig. 3 und 4 schematisch dargestellt. Bei dieser Ausführung ist ein einseitig offenes Rohrstück 9 mit kleinerem Durchmesser in ein ebenfalls einseitig offenes Rohr 10 mit grösserem Durchmesser eingesetzt. Zwischen der Innenwand des Aussenrohres 10 und der Aussenwand des Rohrstückes 9 verbleibt ein Ringraum 11. Im Bereich dieses Ringraumes ist das Rohrstück 9 mit einander gegenüberstehenden Kapillarbohrungen 7 versehen. Diese Kapillarbohrungen bilden ähnlich wie bei der Ausführung nach Fig. 2 die einzige Verbindung zwischen den Rohren 10 und 9. Gemäss Fig. 3 strömt die Voremulsion durch das Rohr 10 in den Ringraum 11 und von dort durch die Kapillarbohrungen in das Rohrstück 9. Da sich die Düsen 7 gegenüberstehen, prallen die austretenden Emulsionsstrahlen im Rohr 9 aufeinander. Hierdurch wird eine besonders gute Dispergierung erreicht. Die homogenisierte Emulsion tritt durch den Auslauf 12 aus dem Strahldispergator aus.

Der Strahldispergator gemäss Fig. 4 ist apparativ identisch mit dem Dispergator nach Fig. 3. Er wird jedoch strömungstechnisch in umgekehrter Richtung betrieben. Die Voremulsion wird hier durch die Öffnung 12 des inneren Rohres 9 zugeführt und strömt dann durch die einander gegenüberliegenden Düsen 7 in den Ringraum 11 zwischen Innenrohr 9 und Aussenrohr 10. Die aus den Düsen 7 austretenden Emulsionsstrahlen treffen auf die Innenwand des Rohres 10 und fliessen von dort zur Ablauföffnung 13. Die Innenwand des Rohres 10 stellt also eine Prallwand für die Emulsionsstrahlen dar. Bei der Ausführung nach Fig. 4 bildet das Rohr 9 das Zulaufrohr und das Rohr 10 das Ablaufrohr des Strahldispergators. Umgekehrt ist bei der Ausführung nach Fig. 3 das Rohr 10 als Zulaufrohr und das Rohr 9 als Ablaufrohr anzusehen. Die Düsen bzw. Kapillarbohrungen 7 sind, gleichmässig über den Umfang verteilt, derart angebracht, dass sich ihre Achsen in einem Punkt auf der Längsachse des Strahldispergators schneiden. Bezüglich ihrer Dimensionierung gilt die oben angegebene Vorschrift.

Die hier beschriebenen Strahldispergatoren sind apparativ sehr einfach und kompakt aufgebaut und lassen sich daher preisgünstig in jeder Werkstatt herstellen. Zu ihrem Betrieb ist ein Druck erforderlich, der in der Grössenordnung von 10 bis 50 bar liegt. Dieser Druck ist wesentlich niedriger als der zum Betrieb eines Hochdruckhomogenisators erforderliche Druck. Aus diesem Grund sind hier einfache Förderpumpen, wie z.B. Zahnradpumpen oder ein- bzw. mehrstufige Exzenterschneckenpumpen, völlig ausreichend. Anstelle der Erzeugung des Betriebsdruckes mit Hilfe von Pumpen können auch die Vorratskessel mit Druckgas beaufschlagt werden. Für Drücke bis zu 15 bar beträgt der Durchsatz pro Einzeldüse ca. 500 Liter pro Stunde, bei 60 bar ca. 1000 Liter pro Stunde. Eine weitere Steigerung des Durchsatzes kann man durch Erhöhung der Düsenzahl und/oder durch Parallelschaltung mehrerer Strahldispergatoren erreichen.

Beispiel 1: Sonnenschutzmilch

In einem normalen Rührwerkskessel 3 wird bei 60 °C die organische Phase (Ölphase), bestehend aus:

| | | |
|---|---|---|
| 200–400 | kg | Emulgator, wie Cetylstearylalkohol und Natrium-Cetylstearylsulfat, + nicht-ionogene Emulgatoren, |
| 1200–1600 | kg | Ölsäuredecylester oder flüssigen Kohlenwasserstoffen, |
| 0,5–2 | kg | Antioxidantien, |
| 50–200 | kg | öllöslicher UV-Filtersubstanz, wie Methyl-phenyl-benzoxazol, |
| 5–20 | kg | Entschäumer, |
| 100–200 | kg | Parfümöl, |

vorgelegt und die kalte wässrige Phase, bestehend aus:

| | | |
|---|---|---|
| 300–800 | kg | Wasser, |
| 100–800 | kg | wasserlösliche Uv-Filtersubstanz, wie Novantisol-Natrium, |

zugegeben. Durch kurzes Rühren entsteht ein Emulsionskonzentrat vom Typ W/O von ca. 40°C. Dieses wird bei 10 bar Druck durch den Strahldispergator 4 in den Lagertank 5 homogenisiert, wo sich

| | | |
|---|---|---|
| 6000–8000 | kg | Wasser, |
| 30–100 | kg | Puffersubstanz, wie Dinatriumhydrogenphosphat, |
| 10–30 | kg | Konservierungsmittel |

befinden. Im Strahldispergator 4 findet die Phasenumkehr statt. Während des Einleitens wird gerührt. Es entstehen so 10 000 kg fertige, flüssige Sonnenschutzemulsion Typ O/W mit einem sehr hohen Dispersitätsgrad.

Beispiel 2: Tagescreme Typ O/W

In einem normalen Kessel 2 wird die organische Phase (Ölphase), bestehend aus:

| | | |
|---|---|---|
| 150–300 | kg | Emulgatorgemisch aus Polyoxyethylen-Stearinsäureester und Sorbitan-Fettsäureester, |
| 400–800 | kg | Cetylstearylalkohol und |
| 600–1200 | kg | Octyldodecanol oder flüssige Kohlenwasserstoffe, |

bei 70°C geschmolzen und auf 40°C eingestellt.

Dann werden die öllöslichen Wirkstoffe und

| | | |
|---|---|---|
| 20–40 | kg | Parfümöl zugegeben. |

In einem anderen Kessel 1 werden in

| | | |
|---|---|---|
| 6000–3000 | kg | Wasser von 45°C, |
| 20–40 | kg | Carboxylvinylpolymerisat |

dispergiert und

| | | |
|---|---|---|
| 300–600 | kg | 1,2-Propylenglykol |

zugegeben. Nach Einstellung auf 40°C werden die wasserlöslichen Werkstoffe zugegeben.

Die Wasserphase wird dann im Rührwerkskessel 3 zur Ölphase gegeben und

| | | |
|---|---|---|
| 100–150 | kg | Natronlauge 10%ig |

zur Neutralisation zugesetzt.

Nach kurzem Rühren wird die noch flüssige Emulsion durch den Strahldispergator 4 bei 10–15 bar Druck homogenisiert. Nach Homogenisierung erstarrt die Creme im Lagerbehälter 5. Es entstehen so 10 000 kg Tagescreme O/W mit einem sehr hohen Dispersitätsgrad.

Beispiel 3: Nachtcreme Typ W/O

In einem normalen Kessel 2 wird die Ölphase aus:

| | | |
|---|---|---|
| 1500–3000 | kg | Salbengrundlage bestehend aus Gemischen wie: Fettsäureestern und Fettalkoholestern (nicht-ionogen) und mineralischen Fetten, |
| 50–150 | kg | Aluminium-Tristearat, |
| 200–400 | kg | Octyldodecanol, |
| 50–150 | kg | mikrokristallinem Wachs, |
| 600–1000 | kg | Kohlenwasserstoffe, |

bei 80°C geschmolzen und auf 75°C eingestellt.

In einem anderen Kessel 1 wird die Wasserphase, bestehend aus:

| | | |
|---|---|---|
| 4500–6000 | kg | Wasser entmineralisiert, |
| 25–75 | kg | Magnesiumsulfat, |
| 200–400 | kg | Glycerin, |
| 10–40 | kg | Konservierungsmittel, |

vorbereitet und auf 45°C eingestellt.

Der Wasserphase werden die wasserlöslichen Wirkstoffe zugesetzt.

Die Wasserphase wird dann im Rührwerkskessel unter Rühren zur Ölphase gegeben. Nach Abkühlung der Rohemulsion auf 45–50°C werden die öllöslichen Wirkstoffe und

| | | |
|---|---|---|
| 40–80 | kg | Parfümöl |

zugegeben. Dadurch entsteht eine Wasser-in-Öl-Emulsion (W/O-Emulsion). Nach Einarbeitung des Parfümöls wird die noch flüssige W/O-Emulsion im Strahldispergator 4 mit 10–15 bar Druck homogenisiert und in den Lagerbehälter 5 abgefüllt, wo sie zur fertigen Creme erstarrt. Dabei bleibt die W/O-Emulsion erhalten, das heisst es findet keine Phasenumkehr statt.

Beispiel 4: Salbenherstellung

In einem gerührten Schmelzkessel 3 wird die organische Phase (Ölphase), bestehend aus:

| | | |
|---|---|---|
| 100–200 | kg | Octyldodecanol, |
| 10–35 | kg | Sorbitan-Fettsäureester oder flüssige Parafine, |
| 20–45 | kg | Wachsgemische, |
| 80–125 | kg | Cetylstearylalkohol, |
| 5–25 | kg | Konservierungsmittel und |
| 5–20 | kg | eines oder mehrerer pharmazeutischer Wirkstoffe (z.B. Azol-, Imidazol- oder Triazolderivate), |

bei 50–75°C aufgeschmolzen und anschliessend wieder auf 35–45°C abgekühlt. Sodann wird die wässrige Phase aus:

| | | |
|---|---|---|
| 550–750 | kg | entkeimten Wasser mit einer Temperatur von 38–45°C, |

in die Ölphase emulgiert (W/O-Emulsion). Der wässrigen Phase können zusätzlich pharmazeutische Wirkstoffe, Emulgatoren und Verdickungsmittel zugesetzt werden. Die so hergestellte Voremulsion wird dann mit einem Vordruck von 10–15 bar und mit einem Stoffstrom von 1000–2000 kg pro Stunde durch den Strahldispergator 4 gepumpt und dadurch homogenisiert. Dabei invertiert die anfängliche W/O-Emulsion in die gewünschte O/W-Emulsion (Öl-in-Wasser-Emulsion). Die fertige Salbe kann entweder direkt in Tuben abgefüllt werden oder man lässt sie vorher in einem Lagertank 5 abkühlen. Alternativ kann die Kühlung auch «in line» durch einen zwischen den Strahldispergator 4 und den Lagertank 5 geschalteten Wärmeaustauscher erfolgen.

**Patentansprüche**

1. Verfahren zur Herstellung von feinteiligen, stabilen, pharmazeutischen oder kosmetischen Dispersionen, dadurch gekennzeichnet, dass eine Voremulsion durch einen Strahldispergator mit den Abmessungen
a) 0,5 bis 0,8 mm Durchmesser und
b) Längen-/Durchmesser-Verhältnis von 1,5 bis 2
gepumpt wird und dabei annähernd die gesamte Pumpenenergie in der Dispergierzone verbraucht wird und die Dispersion als ungerichteter Strahl die Dispergierzone verlässt.

2. Vorrichtung zur Durchführung des Verfahrens nach Anspruch 1 mit einer ersten Emulgiervorrichtung (3) zur Herstellung einer Voremulsion und einer zweiten Emulgiervorrichtung zur Herstellung der fertigen Emulsion, dadurch gekennzeichnet, dass die zweite Emulgiervorrichtung aus einem Strahldispergator (4) mit einer oder mehreren Düsen (7) besteht, deren Durchmesser 0,5 bis 0,8 mm und deren Verhältnis von Länge l zu Durchmesser d 1,5 bis 2 beträgt.

**Claims**

1. Process for the preparation of fine-particled, stable, pharmaceutical or cosmetic dispersions, characterized in that a pre-emulsion is pumped through a jet disperser having the dimensions:
a) 0.5 to 0.8 mm diameter and
b) a length to diameter ratio of 1.5 to 2,
in the course of which almost all the pump energy is consumed in the dispersing zone and the dispersion leaves the dispersing zone as a non-directional stream.

2. Device for carrying out the process according to Claim 1, with a first emulsifying device (3) for the preparation of a pre-emulsion and a second emulsifying device for the preparation of the finished emulsion, characterized in that the second emulsifying device consists of a jet disperser (4) with one or more jets (7) with a diameter of 0.5 to 0.8 mm, and a length l to diameter d ratio of 1.5 to 2.

**Revendications**

1. Procédé de préparation de dispersions pharmaceutiques ou cosmétiques stables et en fines particules, caractérisé en ce que l'on pompe une pré-émulsion dans un appareil de dispersion à jet présentant les dimensions suivantes:
a) 0,5 à 0,8 mm de diamètre et
b) rapport longueur/diamètre de 1,5 à 2,
en consommant ainsi à peu près toute l'énergie de la pompe dans la zone de dispersion d'où la dispersion est évacuée sous forme de jet non dirigé.

2. Appareillage pour la mise en œuvre du procédé selon la revendication 1, comprenant un premier dispositif de mise en émulsion (3) pour la préparation d'une pré-émulsion et un deuxième dispositif de mise en émulsion pour la préparation de l'émulsion finie, caractérisé en ce que le deuxième dispositif de mise en émulsion consiste en un appareil de dispersion à jet (4) à une ou plusieurs tuyères (7) dont le diamètre est de 0,5 à 0,8 mm et le rapport longueur l/diamètre d est de 1,5 à 2.

FIG. 1

FIG. 2

FIG. 3

FIG. 4